# EUROPEAN PATENT APPLICATION

(11) **EP 4 545 955 A1**
(43) Date of publication of application: **30.04.2025**
(21) Application number: 23206423.8
(22) Date of filing: 27.10.2023
(51) Int. Cl.: G01N 24/08, G01N 33/26, G01R 33/30

(54) **NMR SYSTEM FOR ANALYZING AN INSULATING LIQUID**

(71) Applicant: Hitachi Energy Ltd, 8050 Zürich (CH)
(72) Inventor: Cheim, Luiz, Raleigh, 27603 (US); Sbravati, Alan, Raleigh, 27610 (US)
(74) Representative: Epping - Hermann - Fischer

(57) **Abstract**

In an embodiment, the system (100) comprises an electrical apparatus (1) which is configured to be operated by using an insulating liquid (2). The system further comprises an NMR device (3) which is configured to analyze the insulating liquid for one or more specific compounds.

## Description

The present disclosure relates to a system for analyzing an insulating liquid and the use of an NMR device for analyzing an insulating liquid.

One object to be achieved is to provide a system which contributes to a quick and reliable analysis of an insulating liquid. Another object to be achieved is to provide an efficient way to analyze an insulating liquid.

First, the system is specified.

In an embodiment, the system comprises an electrical apparatus which is configured to be operated by using an insulating liquid. The system further comprises an NMR device which is configured to analyze the insulating liquid for one or more specific compounds.

Electrical apparatuses, such as transformers, reactors, phase-shifters, and HVDCs are continuously subjected to a degradation rate, even under regular usage, environmental stresses and loading conditions. Since these are essential pieces of equipment for the backbone of the electrical system of any country, a relevant technological effort is applied to the development of condition-based maintenance and to the condition assessment of such apparatuses. Because they require extremely high availability levels, since a disconnection of one of these apparatuses may lead to an energy outage for a substantial portion of the population and significant length of time, and because the main active components are placed in a sealed tank and immersed in high performance insulating liquids, technologies have been developed over the years to analyze multiple components dissolved in the apparatus insulating liquid, similar to what is done to asses human health by the analysis of compounds found in human blood testing. The most common approach for the condition assessment of such electrical apparatuses is the laboratorial analysis of samples of the insulating liquid, taken periodically.

However, an inherent challenge of such sampling procedures is the long cycle times required to complete each test. Often located in remote locations and/or protected by rigid protocols to control access, the sampling process represents one step of a journey. In order to obtain a valid and representative sample, one or two gallons of the insulating liquids are drained from the equipment, resulting in the need to periodically top up the insulating liquid as well. All of this effort is required for producing a sample of a quarter gallon volume to be sent to a laboratory.

Shipment time generally adds another few days to the process, as does the time required to complete the analysis in the laboratory. Overall, a typical period of two weeks is required between a sample being taken and receipt of the laboratorial report. Should any test result deviate from the expected values, additional tests and samples may be required for more in-depth investigations, triggering the same chain of events again.

The present invention is, inter alia, based on the idea to exploit the NMR technology for the identification of specific compounds in the insulating liquid of an electrical apparatus. Indeed, the NMR technology allows the identification of tiny quantities of compounds in the insulating liquid. NMR devices are meanwhile available in a portable format so that the analysis can be carried out directly at the location of the electrical apparatus. Insulating liquid sampling is no longer required. Thus, a quicker and more reliable analysis of the insulating liquids of an electrical apparatus can be achieved.

An insulating liquid is used for the operation of the electrical apparatus. This means, for example, that an insulating liquid is flowing or circulating through a portion of the electrical apparatus during operation and/or a tank or housing of the electrical apparatus is filled with the insulating liquid during operation. The insulating liquid may be used for cooling and/or insulating purposes. The insulating liquid is, for example, oil. Heat generating elements and/or electrical current transporting elements of the electrical apparatus may be surrounded by the insulating liquid during operation.

The system comprises an NMR device. "NMR" stands for Nuclear Magnetic Resonance. This is a well-known technology. An NMR device comprises a magnet arrangement for producing a magnetic field in a sample region (also called "sample volume"), radio frequency electronics for producing radio frequency signals and a coil for coupling the radio frequency signal into the sample. Moreover, the NMR device may comprise electronics for measuring and/or analyzing the absorption of the radio frequency signal by the sample. The magnet arrangement may be a permanent magnet arrangement or an electric magnet arrangement. For example, the permanent magnet arrangement is a Halbach arrangement.

According to a further embodiment, the electrical apparatus is an electrical transformer or an autotransformer or a reactor or a phase shifter or a HVDC device or similar electrical apparatuses using an insulating liquid whose analysis is used for condition assessment of the equipment.

According to a further embodiment, the NMR device is a mini-NMR device. Particularly, the NMR device is small enough to be installed in or at the electrical apparatus. For example, the NMR device has dimensions which are smaller than 50 cm. That is, the maximum extension of the NMR device in any spatial direction is, for example, less than 50 cm. Particularly, the NMR device may be portable.

Mimi-NMR devices or portable NMR devices, respectively, comprise, for example, a permanent magnet arrangement and an electronic circuit with a silicon chip. The silicon chip may comprise radio frequency electronics for generating the radio frequency signals. It may also comprise the electronics for measuring and analyzing the absorption frequencies of different compounds.

A mini-NMR device or a portable NMR device, respectively, is, for example, described in the papers of Dongwan Ha et al. "Scalable NMR spectroscopy with semiconductor chips", PNAS 2014, Vol. 111, No. 33, pages 11955-11960; or in the paper by Ka-Meng Lei et al. "Portable NMR with Parallelism", Anal. Chem. 2020, 92, 2112-2120. The disclosure content of both papers is hereby incorporated by reference.

According to a further embodiment, the NMR device is external to the electrical apparatus and is fluidically coupled or fluidically couplable to the electrical apparatus. "External" means that the NMR device is not integrated into the electrical apparatus. Rather, it is a separate device. For example, the NMR device is located outside of a housing or tank of the electrical apparatus during operation. Alternatively, it may be placed in the electrical apparatus, for example in a housing thereof, when it is operated.

"Fluidically coupled" means that there is a fluidic connection which allows a portion of the insulating liquid from the electrical apparatus to flow to the sample region of the NMR device.

According to a further embodiment, the system is configured such that the NMR device is supplied with a sample of the insulating liquid from the electrical apparatus in a continuous or intermittent manner. For example, when coupled to the electrical apparatus, a continuous stream of the insulating liquid flows through the sample region of the NMR device. Alternatively, a sample of the insulating liquid can be stored in the sample region of the NMR device for a certain time, for example at least 1 minute, without flowing and, after this time, can be replaced by a further sample of the insulating liquid.

According to a further embodiment, the NMR device is reversibly fluidically couplable to the electrical apparatus. Particularly, the fluidic connection between the NMR device and the electrical apparatus can be repeatedly established and resolved. This enables the NMR device to be fluidically coupled to two or more electrical apparatuses, one after the other, and to analyze the insulating liquid of each of the electrical apparatuses.

According to a further embodiment, the NMR device is simultaneously fluidically coupled or simultaneously fluidically couplable to a plurality of electrical apparatuses. Particularly, when simultaneously coupled to a plurality of NMR devices, a fluidic connection from the NMR device to each of the electrical apparatuses is established. In this way, a sample of the insulating liquid from each of the electrical apparatuses can flow into the sample region of the NMR device.

According to a further embodiment, the NMR device is configured to analyze the insulating liquid of the plurality of electrical apparatuses simultaneously or one after the other. For example, the NMR device comprises a separate sample region for each of the electrical apparatuses to which it is simultaneously fluidically coupled, or samples of two or more electrical apparatuses are mixed in the sample region of the NMR device. Alternatively, when simultaneously fluidically coupled to several electrical apparatuses, samples from different electrical apparatuses are transferred one after the other to the sample region in order to analyze them one after the other.

According to a further embodiment, the NMR-device is configured to analyze the insulating liquid for one or more of the following compounds: dissolved gases, free gases, combustible gases, non-combustible gases, solid and/or liquid insulation aging markers, contaminants, water, dissolved gases, soluble acids, aldehydes, alcohols, oxidation byproducts, peroxides, oxidation inhibitors and additives, sludge, particulate matter.

According to a further embodiment, the system is configured to determine operating parameters of the insulating liquid depending on the compounds analyzed with the help of the NMR-device. For example, the system is configured to analyze and interpret the absorption spectrum of the sample, determine the concentration and types of compounds in the sample and, depending on this, the system determines one or more operating parameters of the electrical apparatus.

According to a further embodiment, the operating parameters are one or more of: Neutralization Index, Dielectric Dissipation Factor, Power Factor, moisture, color, Interfacial Tension, Oxidation Stability, Breakdown Voltage Dissolved Gas Analyses (combustible and non-combustible gases).

According to a further embodiment, the system further comprises a user interface. The user interface is configured to inform a user of the system about the results of the analysis of the insulating liquid with the NMR device. For example, the user interface comprises a display. The system may be configured to show the identified compound(s), optionally also its/their concentration, and/or the operating parameter(s) on the display.

According to a further embodiment, the system is configured to generate an alarm depending on the results of the analysis of the insulating liquid with the NMR-device. Optionally, the system may be configured to generate an expert recommendation for action together with the alarm. By way of example, if it is determined that one or more of the specific compounds are present in the insulating liquid or a concentration of the one or more specific compounds in the insulating liquid exceeds a predetermined threshold, an alarm is generated. The alarm may be a visual indication and/or an acoustic indication. The alarm may indicate to the operator that maintenance must be performed.

Next, the use of the NMR device is specified. All features disclosed in connection with the system are also disclosed for the use and vice versa.

In an embodiment, an NMR device is used for analyzing an insulating liquid for an electrical apparatus. Particularly, the NMR device is used for analyzing the insulating liquid for the presence of one or more specific compounds in the insulating liquid.

According to an embodiment, the analysis of the insulating liquid is carried out on site of the electrical apparatus. For example, the analysis is carried out immediately after extraction of the sample of the insulating liquid from the electrical apparatus. During analysis of the insulating liquid, the NMR device may stay fluidically coupled to the electrical apparatus.

According to an embodiment, the analysis of the insulating liquid is carried out during operation of the electrical apparatus. During operation of the electrical apparatus, the insulting liquid is flowing or circulating through the electrical apparatus and/or the electrical apparatus is at least partially filled with the insulating liquid.

Hereinafter, the system for analyzing an insulating liquid will be explained in more detail with reference to the drawings on the basis of exemplary embodiments. The accompanying figures are included to provide a further understanding. In the figures, elements of the same structure and/or functionality may be referenced by the same reference signs. It is to be understood that the embodiments shown in the figures are illustrative representations and are not necessarily drawn to scale. Insofar as elements or components correspond to one another in terms of their function in different figures, the description thereof is not repeated for each of the following figures. For the sake of clarity, elements might not appear with corresponding reference symbols in all figures.
Figures 1, 3 and 4 show different exemplary embodiments of the system,
Figure 2 shows an exemplary embodiment of an NMR device.

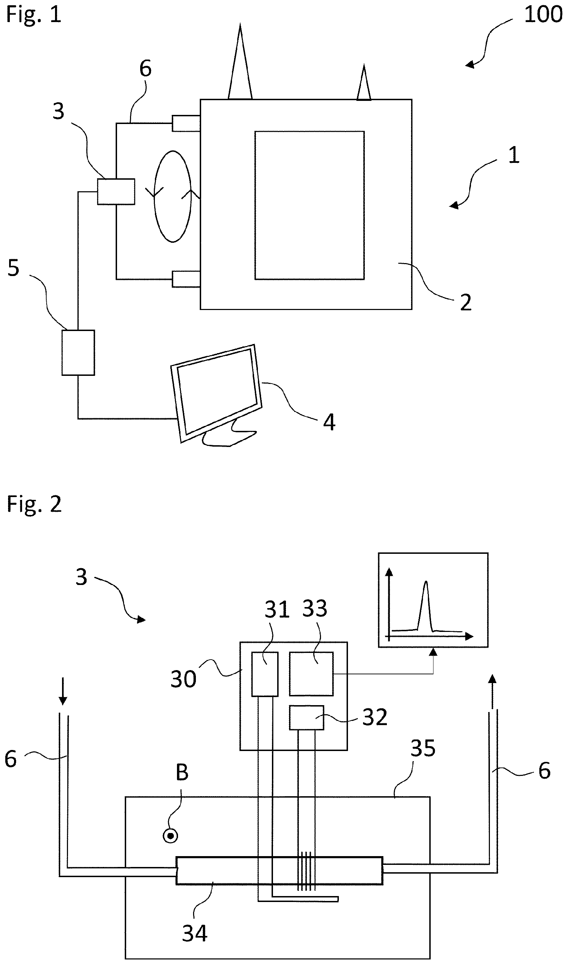

In the first exemplary embodiment of the system for analyzing an insulating liquid according to figure 1, the system 100 comprises an electrical apparatus 1, an NMR device 3, a processor 5 and a user interface 4, namely a display 4. The electrical apparatus 1 is herein realized as a transformer, particularly a high voltage transformer or power transformer, respectively. During operation of the transformer 1, the tank or housing of the transformer 1 is filled with an insulating liquid 2, which might be an oil. This insulating liquid 2 is subject to degradation.

In order to detect such a degradation, the NMR device 3 is fluidically coupled to the transformer 1. This coupling is achieved by a fluidic connection 6, for example a pipe, extending from the transformer 1 to the NMR device 3 and from there back into the transformer 1. In this way, the NMR device 3, namely the sample region thereof, can be provided with a sample of the insulating liquid 2 in a continuous or intermittent manner.

The NMR device 3 is configured to analyze the insulating liquid 2 for one or more specific compounds. The specific compounds could be, for example, dissolved gases, free gases, combustible gases, non-combustible gases, solid and/or liquid insulation aging markers, contaminants, water, soluble acids, aldehydes, alcohols, oxidation byproducts, peroxides, oxidation inhibitors and additives, sludge, particulate matter. Particularly, the NMR device may be configured to detect whether such compounds are present and/or the concentration of such compounds in the insulating liquid 2.

The NMR device 3 is signally connected to the processor 5. The processor 5 is configured to determine operating parameters of the insulating liquid 2 depending on the compounds analyzed with the help of the NMR device 3. For example, depending on the information provided by the NMR device 3, for example the measured absorption spectrum, the processor 5 can determine the Neutralization Index, the Dielectric Dissipation Factor, the Power Factor, moisture, color, the Interfacial Tension, the Oxidation Stability, the Breakdown Voltage and/or Dissolved Gas Analyses. The processor 5 is signally connected to the display 4 so that the operating parameters can be displayed on the display 4.

Figure 2 shows a detailed view of an exemplary embodiment of the NMR device 3 which might be used in the system 100. The NMR device 3 is a mini-NMR device or portable NMR device, respectively. It comprises a magnet arrangement 35, for example a Halbach arrangement of permanent magnets. This magnet arrangement 35 surrounds the sample region 34 into which the sample of the insulating liquid 2 is guided via the fluidic connection 6. A transmitter coil and a receiver coil surround the sample region 34 and are connected to a silicon chip 30 which is an IC chip. The silicon chip 30 comprises a transmitter 31 and a receiver 32 as well as electronics 33 for producing radiofrequency signals and for analyzing the signal received with the help of the receiver 32. Such an analysis is indicated in the diagram of figure 2, showing a peak in the absorption spectrum of the radiofrequency signals indicating the presence of a certain compound in the sample of the insulating liquid 2. The dimensions of the whole NMR device 30 do not, for example, exceed 50 cm in each spatial direction.

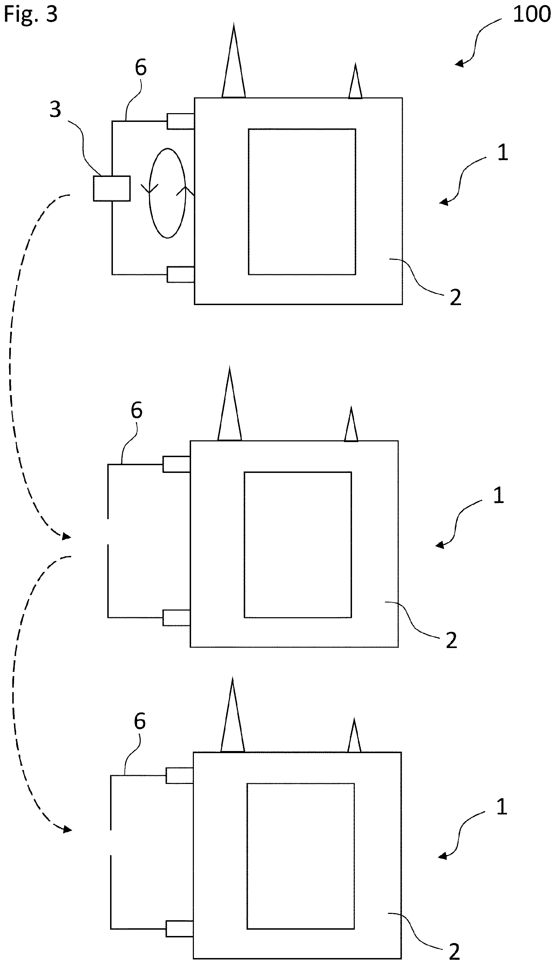

In the exemplary embodiment of figure 3, a system 100 is shown comprising three electrical apparatuses 1, each realized as a transformer. The NMR device 3 is reversibly fluidically couplable to each of the transformers 1. In this way, one NMR device 3 can be coupled to several transformers 1, one after the other, and can be used to analyze the insulating liquid 2 of each of the transformers 1.

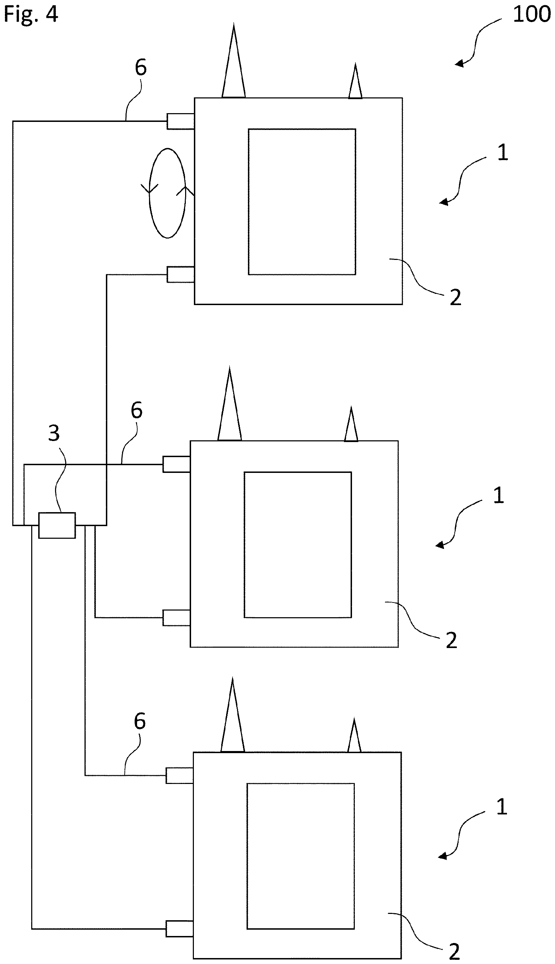

In the exemplary embodiment of figure 4, the system 100 comprises a plurality of electrical apparatuses 1, each realized as a transformer 1. Compared to figure 4, however, the NMR device 3 is now simultaneously fluidically coupled to each of the transformers 1 by a fluidic connection 6. Thus, the NMR device 3 can be used to analyze the insulating liquids 2 of the transformers 1 simultaneously or one after the other.

The embodiments shown in the Figures 1 to 4 as stated represent exemplary embodiments of the system and the NMR-device. Therefore, they do not constitute a complete list of all embodiments according to the system and the NMR-device. Actual systems and NMR-devices may vary from the embodiments shown in terms of arrangements, devices and elements for example.

### Reference signs list:

- 1: electrical apparatus
- 2: insulating liquid
- 3: NMR device
- 4: user interface
- 5: processor
- 6: fluidic connection
- 30: silicon chip
- 31: transmitter
- 32: receiver
- 33: electronics
- 34: sample region / sample volume
- 35: magnet arrangement
- 100: system for analyzing an insulating liquid

## Claims

1. System (100) comprising
- an electrical apparatus (1) which is configured to be operated by using an insulating liquid (2),
- a NMR device (3) which is configured to analyze the insulating liquid (2) for one or more specific compounds.

2. System (100) according to claim 1, wherein
- the electrical apparatus (1) is an electrical transformer or an autotransformer or a reactor or a phase shifter or a HVDC device.

3. System (100) according to claim 1 or 2, wherein
- the NMR device (3) is a mini-NMR device (3) with dimensions smaller than 50 cm.

4. System (100) according to any one of the preceding claims, wherein
- the NMR-device (3) is external to the electrical apparatus (1) and is fluidically coupled or fluidically couplable to the electrical apparatus (1).

5. System (100) according to any one of the preceding claims, wherein
- the system (100) is configured such that the NMR-device (3) is supplied with a sample of the insulating liquid (2) from the electrical apparatus (1) in a continuous or intermittent manner.

6. System (100) according to any one of the preceding claims, wherein
- the NMR-device (3) is reversibly fluidically couplable to the electrical apparatus (1) in order to enable the NMR-device (3) to be fluidically coupled to two or more electrical apparatuses (1), one after the other, and to analyze the insulating liquid (2) of the electrical apparatuses (1).

7. System (100) according to any one of the preceding claims, wherein
- the NMR-device (3) is simultaneously fluidically coupled or simultaneously fluidically couplable to a plurality of electrical apparatuses (1),
- the NMR-device (3) is configured to analyze the insulating liquid (2) of the plurality of electrical apparatuses (1) simultaneously or one after the other.

8. System (100) according to any one of the preceding claims, wherein
- the NMR-device (3) is configured to analyze the insulating liquid (2) for one or more of the following compounds: dissolved gases, free gases, combustible gases, non-combustible gases, solid and/or liquid insulation aging markers, contaminants, water, soluble acids, aldehydes, alcohols, oxidation byproducts, peroxides, oxidation inhibitors and additives, sludge, particulate matter.

9. System (100) according to any one of the preceding claims, wherein
- the system (100) is configured to determine operating parameters of the insulating liquid (2) depending on the compounds analyzed with the help of the NMR-device (3).

10. System (100) according to claim 9, wherein
- the operating parameters are one or more of: Neutralization Index, Dielectric Dissipation Factor, Power Factor, moisture, color, Interfacial Tension, Oxidation Stability, Breakdown Voltage and Dissolved Gas Analyses.

11. System (100) according to any one of the preceding claims, further comprising
- a user interface (4) configured to inform a user of the system (100) about the results of the analysis of the insulating liquid (2) with the NMR-device (3).

12. System (100) according to any one of the preceding claims, wherein
- the system (100) is configured to generate an alarm with expert recommendation for action, depending on the results of the analysis of the insulating liquid (2) with the NMR-device (3) .

13. Use of an NMR-device (3) for analyzing an insulating liquid (2) for an electrical apparatus (3).

14. Use of the NMR-device (3) according to claim 13, wherein
- the analysis of the insulating liquid (2) is carried out on site of the electrical apparatus (1).

15. Use of the NMR-device (3) according to claim 13 or 14, wherein
- the analysis of the insulating liquid (2) is carried out during operation of the electrical apparatus (1).
